# EUROPEAN PATENT APPLICATION

(11) **EP 4 567 003 A1**
(43) Date of publication of application: **11.06.2025**
(21) Application number: 23214847.8
(22) Date of filing: 07.12.2023
(51) Int. Cl.: C02F 1/46, A61C 17/02, A61L 2/14, H05H 1/24, C02F 103/02

(54) **APPARATUS AND METHOD FOR GENERATING PLASMA ACTIVATED WATER**

(71) Applicant: Koninklijke Philips N.V., 5656 AG Eindhoven (NL)
(72) Inventor: VISSER, Cornelis, Eindhoven (NL); THUMMA, Kiran Kumar, Eindhoven (NL); BORN, Matthias, Eindhoven (NL)
(74) Representative: Philips Intellectual Property & Standards

(57) **Abstract**

The invention concerns an apparatus for generating plasma activated water, comprising a chamber partially filled with water, an humidifier arranged inside the chamber and configured to generate a relative humidity larger than 85 percent from the water inside the chamber, a plasma generating element arranged and configured for generating plasma inside the chamber, such that the generated plasma is able to interact with the humidified air inside the chamber. Further, the invention concerns a method for generating plasma activated water.

## Description

### FIELD OF THE INVENTION

The present invention relates to the field of generating of plasma activated water. In particular, the present invention relates to the field of plasma generation in a humidified environment for generating activated reactive species in water.

### BACKGROUND OF THE INVENTION

For generating plasma activated water, water is treated with plasma using specific parameters such as plasma forming voltage, carrier gas, temperature, pulses. Plasma-activated water (PAW) is water that has changed properties as a result of plasma treatment and is used among other things for disinfection. The plasma process produces reactive oxygen and nitrogen species, some of which dissolve in the water and react with the water to form other species. PAW has an antimicrobial effect. Plasma-activated water, which is generated using only electricity, water and air, is considered a favorable disinfectant solution whose biocidal effect lasts from a few minutes to several months, depending on the microorganisms. Devices for generating PAW may humidify an air flow, wherein a so-called water bubbler is used. Humidity is picked up by bubbling the air-flow through water. These bubblers typically generate a relative humidity of 60 to 75 percent. For achieving a higher relative humidity the water has to be heated, or heating the air flow.

### SUMMARY OF THE INVENTION

There may therefore be a need for an improved apparatus for generating plasma activated water, wherein in a simple and fast way the activated water is generated without the need of additional elements such as the heating of the water and/or the air flow.

An object of the invention is to provide an improved apparatus for generating plasma activated water which comprises a high efficiency in providing activated species and which is able to produce the plasma activated water with low energy consumption.

The object of the present invention is solved by the subject matter of the independent claims where further embodiments are incorporated in the dependent claims.

According to a first aspect of the invention, an apparatus for generating plasma activated water is described. The apparatus comprises a chamber partially filled with water, an humidifier arranged inside the chamber and configured to generate a relative humidity larger than 85 percent from the water inside the chamber, a plasma generating element arranged and configured for generating plasma inside the chamber, such that the generated plasma is able to interact with the humidified air inside the chamber.

In other words, the present aspect of the invention relates to an activation apparatus for producing plasma activated water were the plasma activated water may be used to inactivate bacteria and viruses. The term "activating" may particular denote modifying at least one physical, biological, or chemical characteristic of a material or substance in such a manner that the material or substance is brought into a state to be able to generate a desired influence to the surrounding environment. Hence the activated water may be used to destroy specific cells. With the present invention a relative high humidity larger than 85 percent can be generated without the need of heating and without the need for temperature control. Therefore, a simpler apparatus can be designed which may be used by untrained users, such as normal consumers. Hence, the herein described apparatus may be a simpler and compact plasma activated water generating device with high relative humidity and low energy consumption. Inside the apparatus the plasma generated inside the chamber and also the water is humidified inside the chamber.

In particular, the plasma may be generated in the mist of the air, where the air is highly saturated. Therefore, the chamber of the apparatus is not fully filled with water. The amount of water may depend on the chosen humidifier and the lever needed for the humidifier to produce mist from the water. The generated activated species diffuse into the water. The higher the humidity the better and the more activated species may diffuse into the water and may create plasma activated water. Therefore, there exist a need for a high relative humidity above 85 percent inside the chamber.

In the present invention, the term "relative humidity" shall be understood to describe a present state of absolute humidity relative to a maximum humidity given the same temperature. In particular, the relevant relative humidity is the relative humidity inside the chamber, which needs to be high enough for providing sufficient humidified air for the plasma interaction with the humidified air. When using an air bubbler the relative humidity is less than 85 percent.

In the context of the present invention, the term "plasma generating device" shall be understood to describe any device which is able the generate plasma and which device may be arrangeable and attachable to the chamber as described with the different exemplary embodiments herein. The plasma generating device may be configured to generate plasma by high electrical current discharge in the air. This allows the generation of plasma in the air of the chamber, which is used for generating plasma activated water. The plasma generating element may not necessarily be fully arranged inside the chamber. It might be sufficient that at least the parts used for generating the plasma or the part from which the plasma extends may be arranged inside the chamber.

According to an exemplary embodiment of the invention, the humidifier may be configured to generate cold humidified air, such as an ultrasound piezo element or an impeller humidifier. For generating cold humidified air the water and/or the air must not be heated, such that additional heating devices may be omitted. The ultra sound piezo element may be any element which is able to produce ultra-sonic waves which in turn may be used to evaporate water, in particular inside the chamber of the present apparatus. The term "ultrasonic waves" shall be understood to describe frequencies above the normal human hearing, generally to be at 20 kHz to 2 MHz and above, but also extended down to the 5 kHz to 20 kHz range in certain processing applications. The piezo element may be an ultrasound transducer which may be able to produce mist of very fine water droplets. This mist may be combined with the air flow inside the chamber, for example airflow due to diffusion and/or due to the movement of the humidifier, in particular of the piezo element, where the fine droplets evaporate.

On the other hand, an impeller humidifier may use a rotating disc to gently massage water onto a diffuser, which then turns the water into tiny droplets and flows in the air to moisturize.

According to an exemplary embodiment of the invention, the chamber may comprise a first portion and a second portion separated by a wall, wherein the humidifier may be arranged in the first portion, and wherein the plasma generating element may be arranged in the second portion. The arrangement inside portions separated from each other according to this embodiment may allow to space apart the plasma generation element from the humidifier. Further, the water used for humidifying the air may be separated from the plasma activated water, which may allow a better extraction of the plasma activated water of apparatus for the further use.

According to an exemplary embodiment of the invention, the humidified air may be generated in the first portion of the chamber and may be provided to the second portion, such that the plasma generated by the plasma generating element in the second portion can interact with the humidified air provided in the second portion. In the first portion the air is humidified and due to a concentration gradient of the humidified air, the humidified air may be transported/diffused into the second portion. Further, the concentration of the humidified air, i.e. the relative humidity, in the second portion may be increased such that a high concentration of relative humidity can be provided to the plasma generating device inside the second portion.

According to an exemplary embodiment of the invention, the humidifier may be configured to generate a relative humidity larger than 90 percent from the water inside the chamber. The higher the relative humidity the more efficient the generating of activated relative species may be carried out by the apparatus. When more activated reactive species could be generated this may result in a plasma activated water comprising more enriched reactive species.

According to an exemplary embodiment of the invention, the humidifier may generate the humidified air from the water inside the chamber without requiring the use of heating the water inside the chamber. According to this embodiment the apparatus does not need any device for heating the air, the water or both. Hence, any combination of heating and temperature control may be obsolete. Even without the necessity of a heating device the apparatus is able to produce humidified air inside the chamber which higher than 85 percent. As further elements may be omitted the relative humidity can be provided in a sustainable manner because less energy is necessary in comparison to apparatuses using a heating device and/or temperature control.

According to an exemplary embodiment of the invention, when the plasma interacts with the humidified air activated water is generated comprising reactive species. In particular, the activated reactive species are generated in the humidified air and they diffuse into the water, such that the water comprises the activated reactive species. Accordingly, the plasma activated water may be generated.

According to an exemplary embodiment of the invention, the apparatus may further comprise a spraying element arranged at the chamber and may be configured to provide a path for the water from the chamber to the outside of the chamber by spraying the water outside of the chamber. The path may connect the interior of the chamber with the outer environment, such that the plasma activated water can be released. The spraying element may be used to spray and/or evaporate the plasma activated water to the outside for the further use. For example the apparatus as described with the exemplary embodiments herein may be used as a stand-alone device for producing plasma activated water and for using the plasma activated water by applying it with the spraying element to surfaces or any elements which need to be decontaminated by the plasma activated device. On the other hand the apparatus according to the exemplary embodiments described herein may be used as a tank holding the plasma activated water and the spraying element is used to transfer the plasma activated water to a container for its further use.

According to an exemplary embodiment of the invention, the plasma generating element may comprise an electrode and may be configured to generate a unipolar corona discharge for generating the plasma. The term "corona discharge" shall be understood to describe an electrical discharge in a nonconducting medium, such as air. This discharge is able to produce plasma, which in turn may interact with the humidified air for producing the activated reactive species.

According to an exemplary embodiment of the invention, the plasma generating element may comprise a plasma jet for generating the plasma, wherein the plasma jet may be arranged at the chamber such that the plasma is generated inside the chamber. For instance, the plasma jet may be partially arranged inside the chamber or may be arranged completely inside the chamber. In particular, at least the components of the plasma jet used for generating the plasma should be arranged inside the chamber, such that the plasma is generated inside the chamber. When the plasma is generated inside the chamber the plasma can interact with the humidified air and/or the water for generating activated reactive species.

According to an exemplary embodiment of the invention, the humidifier may be configured for mixing the humidified air and the water inside the chamber by generating water movements and water droplets. For instance, the humidifier itself is able to move the water and/or the air inside chamber. For example an ultrasonic humidifier may generate water movement as a result of the ultrasonic transducer which creates strong water movements and water droplets, such that the air and water are mixed. This in turn facilitate the diffusion and transfer of the active species produced by the plasma.

According to an exemplary embodiment of the invention, the apparatus may further comprise a mixing element configured for mixing the humidified air and the water inside chamber. Any element or component which may be able to mix water and/or the air may be used as the mixing element. This mixing element may be arranged inside the chamber. For example a rotor may be used for mixing and moving the water. A rotor may also be used for mixing and moving the air and/or the humidified air. The mixing element may be arranged in the area of the chamber comprising the water, for mixing the water. On the other hand the mixing element may be arranged on the area comprising the air and/or the humidified air for mixing the respective air. The mixing element may facilitate the diffusion and transfer of the active species produced by the plasma.

According to a second aspect of the invention, an oral healthcare device comprising the apparatus according to any of the herein described embodiments is described, wherein the apparatus is configured for generating plasma activated water for oral care. In particular, the oral healthcare device may be a consumer device usable by untrained people for generating plasma activated water which may be used as a mouthwash for neutralizing bacteria or viruses in the mouth and/or between the teeth. The oral healthcare device may be used for generating plasma activated water by the user himself.

According to a third aspect of the invention, a method for generating plasma activated water is described. The method comprising the steps of filling a chamber partially with water, generating a relative humidity larger than 85 percent using a humidifier and the water inside the chamber, generating a plasma inside the chamber using a plasma generating element, such that the plasma is able to interact with the humidified air inside the chamber. The above mentioned steps need not necessarily be carried out in the order as mentioned, hence the order may differ or further steps or sub steps may be added. The method may further comprise the step of providing a chamber before providing water into the chamber. Further, the method may comprise providing a plasma generating element to the chamber, such that the plasma may be generated inside the chamber before the step of generating plasma.

According to a further embodiment of the invention, the step of generating the relative humidity comprises generating cold humidified air using an ultrasound piezo element or an impeller humidifier. In other words, the generated humidified air is non heated air. The step may comprise the generating of humidified air by the ultrasound humidifier, the piezo element or by the impeller humidifier, a combination of both humidifying technologies may also be possible.

According to a further embodiment of the invention, the method may further comprise the step of generating, when the plasma interacts with the humidified air, activated water comprising reactive species.

It has to be noted that embodiments of the invention are described with reference to different subject matters. In particular, some embodiments are described with reference to method type claims whereas other embodiments are described with reference to the apparatus type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

### BRIEF DESCRIPTION OF THE DRAWINGS

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.
Fig 1 illustrates schematically an apparatus for generating plasma activated water according to an exemplary embodiment of the invention.
Fig. 2 illustrates schematically an apparatus for generating plasma activated water according to a further exemplary embodiment of the invention.
Fig. 3 illustrates schematically an apparatus for generating plasma activated water according to a further exemplary embodiment of the invention.

### DETAILED DESCRIPTION OF EMBODIMENTS

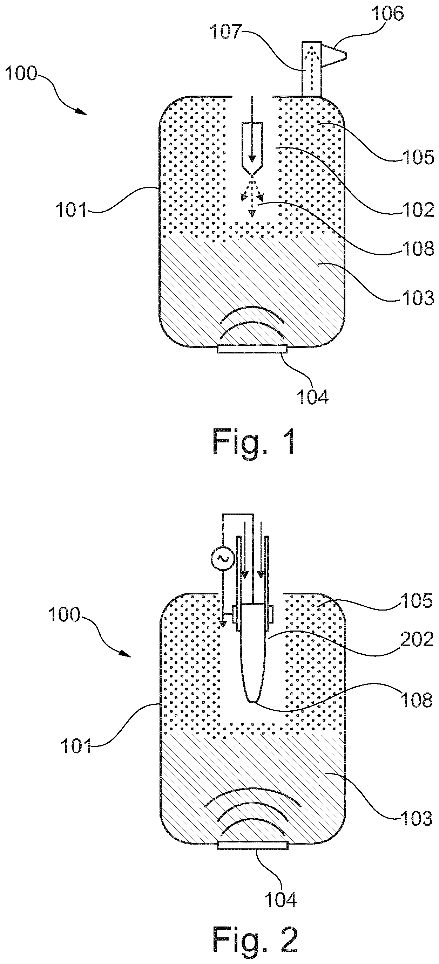

Fig 1 illustrates schematically an apparatus 100 for generating plasma activated water according to an exemplary embodiment of the invention. The apparatus 100 comprises a chamber 101 partially filled with water 103. Further, the apparatus 100 comprises an humidifier 104 arranged inside the chamber 101 and configured to generate a relative humidity 105 larger than 85 percent from the water 103 inside the chamber 101. The humidifier 104 may be arranged at the bottom of the chamber 101. However, the humidifier may also be arranged at a side wall of the chamber 101, as long as the humidifier 104 is able to generate mist for humidifying the air. Accordingly, the humidifier 104 may be at least arranged in such a manner that it is in contact with the water 103 inside the chamber 101. Furthermore, the apparatus 100 comprises a plasma generating element 102 arranged and configured for generating plasma 108 inside the chamber 101, such that the generated plasma 108 is able to interact with the humidified air 105 inside the chamber 101. The humidifier 104 generates the humidified air 105 from the water 103 inside the chamber 101 without requiring the use of heating the water 105 inside the chamber 101. The plasma 108 generated by the plasma generating element 102 interacts with the humidified air 105 and plasma activated water is generated comprising reactive species. The apparatus 100 further comprises a spraying element 106 arranged at the chamber 101 and configured to provide a path 107 for the water from the chamber 101 to the outside of the chamber by spraying the water outside of the chamber 101. For example, the spraying element may be a nozzle configured to spray the activated water outside the chamber 101.

The plasma generating element 102 comprises an electrode and is configured to generate a unipolar corona discharge for generating the plasma 108. As can be seen in Fig. 1 the plasma generating element is arranged on the top of the chamber 101, above the water 103 and above the humidifier. The humidifier 104 is configured for mixing the humidified air and the water inside the chamber 1101 by generating water movements and water droplets. For example, when a piezo element is used this piezo element may generate water movements which may mix the water 103 and the humidified air 105.

Fig. 2 illustrates schematically an apparatus 100 for generating plasma activated water according to a further exemplary embodiment of the invention. In this embodiment the apparatus 100 differs from the apparatus in Fig. 1 in that the plasma generating element is a plasma jet 202 which generates the plasma 108. The plasma jet comprises electrodes for generating a high current for generating the plasma 108. The plasma jet 202 is arranged partially inside the chamber 101. In particular, the plasma jet 202 may at least be arranged in such a manner that the plasma jet generates the plasma inside the chamber. For example at least one opening may be arranged, for example at the top of the chamber, through which a tip of the plasma jet extends inside the chamber 101. Hence, at least the element of the plasma jet 202 from which the plasma 108 is produced may be arranged inside the chamber 101.

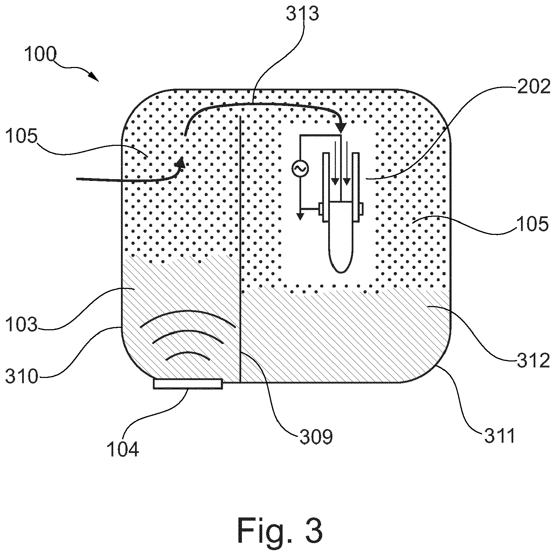

Fig. 3 illustrates schematically an apparatus 100 for generating plasma activated water according to a further exemplary embodiment of the invention. The apparatus 100 as illustrated in Fig. 3 comprises the same features as the apparatus 100 described with Fig. 2. Additionally, the apparatus of Fig. 3 comprises a wall 309 separating the chamber 101 into a first portion 310 and a second portion 312. Hence, the chamber 101 comprises a first portion 310 and a second portion 311 separated by the wall 309, wherein the humidifier 104 is arranged in the first portion 310, the plasma generating element 202 is arranged in the second portion 311. The humidified air 105 is generated in the first portion 310 and is provided to the second portion 311, such that the plasma 108 generated by the plasma generating element 202 in the second portion 311 can interact with the humidified air 105 provided in the second portion 311. The humidified air may diffuse along a concentration gradient along the humidified air path 313 from the first portion 310 into the second portion 311 of the chamber 101. The plasma activated water 312 is generated inside the second portion 311 and is therefore separated from the water 103 used for humidifying the air. The wall 309 separating the two portions 310, 311 partially extend inside chamber 101, wherein a path 313 for the humidified air is left open, such that the humidified air 105 can diffuse into the second portion 311.

### LIST OF REFERENCE SIGNS:

- 100: apparatus
- 101: chamber
- 102: plasma generating element
- 103: water
- 104: humidifier
- 105: humidified air
- 106: nozzle
- 107: spraying path
- 108: plasma
- 202: plasma jet
- 309: wall
- 310: first portion
- 311: second portion
- 312: plasma activated water
- 313: humidified air path

## Claims

1. An apparatus for generating plasma activated water, comprising
a chamber partially filled with water,
a humidifier arranged inside the chamber and configured to generate a relative humidity larger than 85 percent from the water inside the chamber,
a plasma generating element arranged and configured for generating plasma inside the chamber, such that the generated plasma is able to interact with the humidified air inside the chamber.

2. The apparatus according to claim 1,
wherein the humidifier is configured to generate cold humidified air, such as an ultrasound piezo element or an impeller humidifier.

3. The apparatus according to claim 1 or 2,
wherein the chamber comprises a first portion and a second portion separated by a wall,
wherein the humidifier is arranged in the first portion,
wherein the plasma generating element is arranged in the second portion.

4. The apparatus according to claim 3,
wherein the humidified air is generated in the first portion and is provided to the second portion, such that the plasma generated by the plasma generating element in the second portion can interact with the humidified air provided in the second portion.

5. The apparatus according to any of the preceding claims,
wherein the humidifier is configured to generate a relative humidity larger than 90 percent from the water inside the chamber.

6. The apparatus according to any of the preceding claims,
wherein the humidifier generates the humidified air from the water inside the chamber without requiring the use of heating the water inside the chamber.

7. The apparatus according to any of the preceding claims,
wherein, when the plasma interacts with the humidified air activated water is generated comprising reactive species.

8. The apparatus according to any of the preceding claims,
wherein the apparatus further comprises a spraying element arranged at the chamber and configured to provide a path for the water from the chamber to the outside of the chamber by spraying the water outside of the chamber.

9. The apparatus according to any of the preceding claims,
wherein the plasma generating element comprises an electrode and is configured to generate a unipolar corona discharge for generating the plasma.

10. The apparatus according to any of the preceding claims 1 to 8,
wherein the plasma generating element comprises a plasma jet for generating the plasma, wherein the plasma jet is arranged at the chamber such that the plasma is generated inside the chamber.

11. The apparatus according to any of the preceding claims,
wherein the humidifier is configured for mixing the humidified air and the water inside the chamber by generating water movements and water droplets.

12. The apparatus according to any of the preceding claims,
further comprising a mixing element configured for mixing the humidified air and the water inside chamber.

13. An oral healthcare device comprising the apparatus according to any of the preceding claims, configured for generating plasma activated water for oral care.

14. Method for generating plasma activated water, the method comprising the following steps of
filling a chamber partially with water,
generating a relative humidity larger than 85 percent using a humidifier and the water inside the chamber,
generating a plasma inside the chamber using a plasma generating element, such that the plasma is able to interact with the humidified air inside the chamber.

15. The method according to claim 14,
wherein the step of generating the relative humidity comprises generating cold humidified air using an ultrasound piezo element or an impeller humidifier,
further comprising the step of
generating, when the plasma interacts with the humidified air, activated water comprising reactive species.
